# EUROPEAN PATENT APPLICATION

(11) **EP 4 483 912 A1**
(43) Date of publication of application: **01.01.2025**
(21) Application number: 23770859.9
(22) Date of filing: 15.03.2023
(51) Int. Cl.: A61L 27/20, A61K 35/30, A61K 47/36, A61L 27/38, C12N 5/071

(54) **TRANSPLANTATION MEDIUM**

(30) Priority: 16.03.2022 JP 2022041536
(71) Applicant: Sumitomo Pharma Co., Ltd., Osaka-shi, Osaka 541-0045 (JP)
(72) Inventor: KAMEI Tatsuya, Kobe-shi, Hyogo 650-0047 (JP); MANABE Koichiro, Tokyo 103-6012 (JP); TAKAMURA Naoki, Kobe-shi, Hyogo 650-0047 (JP); WATANABE Hitoshi, Kobe-shi, Hyogo 650-0047 (JP); KAGIHIRO Masashi, Suita-shi, Osaka 564-0053 (JP); KASAHARA Satomi, Osaka-shi, Osaka 554-0022 (JP)
(74) Representative: Vossius & Partner Patentanwälte Rechtsanwälte mbB
(86) International application number: PCT/JP2023/010178
(87) International publication number: WO 2023/176906

(57) **Abstract**

A transplantation medium for subretinal transplantation of a retinal pigment epithelial cell contains hyaluronic acid and a pharmaceutically acceptable aqueous liquid, in which the viscosity of the transplantation medium at a shear rate of 2 (1/s) at 25°C is 1.5 to 4 mPa·s.

## Description

### Technical Field

The present invention relates to a transplantation medium, particularly a transplantation medium for subretinal transplantation of retinal pigment epithelial cells.

### Background Art

Retinal pigment epithelial (RPE) cells are pigment epithelial cells present in the outermost layer of retina and play an important role, for example, in photoreceptor maintenance, such as phagocytosis of photoreceptor outer segments and recycling of visual substances. Age-related macular degeneration caused by abnormality of retinal pigment epithelial cells due to aging and other factors is an eye disease that causes loss of central vision or blindness, and it has been desired to develop an effective treatment for the disease. Recently, cell transplantation therapy, which supplements or substitutes retinal pigment epithelial cells, has attracted a lot of attention as a novel treatment for age-related macular degeneration, and it has been expected to utilize retinal pigment epithelial cells as grafting materials for cell therapy. Several methods of inducing differentiation into retinal pigment epithelial cells using human pluripotent stem cells are known (Patent Literature 1),

A general method of transplanting RPE cells into subretinal space of the eyeball involves, for example, aspirating a suspension of RPE cells to the inside of Surflo(R) I.V catheter, then inserting Surflo(R) I.V catheter into the subretinal space of the eyeball to eject the RPE cells from the tip of Surflo(R) I.V catheterto the subretinal space (Non Patent Literatures 1 and 2). Since RPE cells must be accurately implanted and engrafted into diseased retinal tissue in need of repair, there has been a need for a medium suitable for RPE cell transplantation surgery.

Non Patent Literature 3 describes screening and optimization of a medium for preserving RPE stem cells before transplantation, and influence on the survival, attachment, distribution, proliferation, and differentiation potency into RPE cells, of RPE stem cells has been examined as to each medium. It also describes that in the case of using a 0.2% hyaluronic acid solution as a preservation medium for RPE stem cells, favorable distribution and proliferation of RPE stem cells as well as cobblestone formation unique to matured RPE cells and expression of RPE cell markers have been found to be promoted after preservation for 96 hours. However, Non Patent Literature 3 does not describe an effect in the case of actual use in transplantation surgery. Patent Literature 2 discloses a device for subretinally transplanting an implant (retina cell graft) and describes the use of an aqueous hyaluronic acid solution therein, does not describe any specific retinal tissue or the concentration of hyaluronic acid. It has been further reported that retinal detachment occurs in the case of subretinally injecting hyaluronic acid (Non Patent Literature 4). As described above, a medium used in transplantation of RPE cells has not yet been established.

### Citation List

### Patent Literature

Patent Literature 1: WO2017/043605
Patent Literature 2: Japanese Unexamined Patent Publication No. 2005-517468

### Non Patent Literature

Non Patent Literature 1: Schwartz SD et al. "Embryonic stem cell trials for macular degeneration: a preliminary report." Lancet. 2012;379(9817):713-720
Non Patent Literature 2: Sugita S et al. "HLA-Matched Allogeneic iPS Cells-Derived RPE Transplantation for Macular Degeneration." JClin Med. 2020;9(7):2217.
Non Patent Literature 3: Y. Tian et al., "Screening and optimization of potential injection vehicles for storage of retinal pigment epithelial stem cell before transplantation", J Tissue Eng Regen Med. 2019;13(1): p76-86.
Non Patent Literature 4: T. Nakazawa, et al., "Tumor Necrosis Factor-a Mediates Photoreceptor Death in a Rodent Model of Retinal Detachment", Investigative Ophthalmology & Visual Science, March 2011, Vol.52, No.3, p1384-1391

### Summary of Invention

### Technical Problem

The present inventors have investigated a suitable transplantation medium for the transplantation of RPE cells and then found a problem that when transplantation is conducted in a state of an RPE cell suspension, an epiretinal membrane (ERM), which is considered to be due to cell leakage to the vitreous body, formed, and localized engraftment occurred only in a part of an injection site. Furthermore, the present inventors have found a problem that prolonged retinal detachment occurs when the viscosity is increased to improve engraftment by preventing cell leakage.

Accordingly, an object of the present invention is to provide a retinal pigment epithelial cell transplantation medium and transplantation composition capable of suppressing cell leakage into the vitreous body and prolonged retinal detachment after subretinal transplantation.

### Solution to Problem

As a result of extensive studies on a transplantation medium, it has been found that a medium having a specific viscosity is the best medium for RPE cell transplantation.

In other words, the invention provides the following:
[1] A transplantation medium for subretinal transplantation of retinal pigment epithelial cells, the transplantation medium comprising:
   hyaluronic acid and a pharmaceutically acceptable aqueous liquid,
   wherein a viscosity at a shear rate of 2 (1/s) at 25°C is 1.5 to 4 mPa·s.
[2] The transplantation medium according to [1], wherein the viscosity at a shear rate of 2 (1/s) at 25°C is 2 to 3 mPa·s.
[3] The transplantation medium according to [1] or [2], wherein the viscosity at a shear rate of 1000 (1/s) at 25°C is 3 mPa·s or less, and a difference in viscosity between the viscosity at a shear rate of 1 (1/s) and the viscosity at a shear rate of 10 (1/s) is 0.5 to 1.5 mPa·s.
[4] The transplantation medium according to any one of [1] to [3], wherein pH is 7.0 to 7.5.
[5] The transplantation medium according to any one of [1] to [4], wherein the pharmaceutically acceptable aqueous liquid is a balanced salt solution.
[6] The transplantation medium according to any one of [1] to [5], which is free of an antibacterial agent and a preservative.
[7] The transplantation medium according to any one of [1] to [6], which is free of chondroitin sulfate.
[8] The transplantation medium according to any one of [1] to [7], wherein in the transplantation, the vitreous body is replaced with air after subretinal transplantation.
[9] A transplantation composition comprising:
   retinal pigment epithelial cells for transplantation; and
   the transplantation medium according to any one of [1] to [8].
[10] The transplantation composition according to [9], wherein the retinal pigment epithelial cells for transplantation are comprised at a concentration of 1 × 10⁶ cells/mL to 5 × 10⁷ cells/mL.
[11] The transplantation composition according to [9] or [10], wherein a volume per transplantation is 10 µL to 100 µL.
[12] Use of the transplantation medium according to any one of [1] to [8], for subretinal transplantation of retinal pigment epithelial cells.
[13] The transplantation medium according to any one of [1] to [8], for use in subretinal transplantation of retinal pigment epithelial cells.
[14] A therapeutic agent for a disease based on a disorder of retinal pigment epithelial cells, the therapeutic agent comprising:
   the transplantation composition according to any one of [9] to [11].
[15] A method for treating a disease based on a disorder of retinal pigment epithelial cells, comprising:
   a step of administering to a patient in need thereof an effective amount of the transplantation composition according to any one of [9] to [11] or an effective amount of the therapeutic agent according to [14].

### Advantageous Effects of Invention

The transplantation medium and transplantation composition of the present invention can suppress cell leakage into the vitreous body and prolonged retinal detachment after subretinal transplantation, thus efficiently engrafting retinal pigment epithelial cells transplanted to a desired region while reducing side effects.

### Brief Description of Drawings

[Figure 1] Figure 1 is a schematic view showing a procedure for subretinal injection of RPE cells by a transvitreal cavity method.
[Figure 2] Figure 2 shows fluorescence images of RPE cells subretinally injected to an enucleated porcine eye in Example 1.
[Figure 3] Figure 3 shows low-magnification fluorescent immunostaining images of the retina including a representative injection site in the fluorescent immunostaining of Example 2.
[Figure 4] Figure 4 shows low-magnification fluorescent immunostaining images of the retina including a representative injection site in the fluorescent immunostaining of Example 2.
[Figure 5] Figure 5 shows high-magnification fluorescent immunostaining images of the retina including a representative injection site in the fluorescent immunostaining of Example 2.

### Description of Embodiments

### 1. Definition

As used herein, the term "retinal pigment epithelial (RPE) cell" refers to an epithelial cell present on the outside the neural retina in retina *in vivo.* Whether the cell is a retinal pigment epithelial cell can be easily confirmed by those skilled in the art based on, for example, the expression of cell markers (RPE65, MITF, CRALBP, MERTK, BEST1, TTR etc.), presence of melanin granules (brown-black), intercellular tight junction, and characteristic polygonal or cobblestone-like cell morphology. Whether the cell has the function of a retinal pigment epithelial cell can be easily confirmed by, for example, the secretory capacity of cytokines such as VEGF and PEDF. In one aspect, the retinal pigment epithelial cells are RPE65-positive cells, MITF-positive cells, or RPE65-positive and MITF-positive cells.

Although RPE cells to be transplanted herein are not particularly limited, it is preferable that the RPE cells be derived from rodents, carnivores, or primates, especially RPE cells derived from humans. It is also preferable that the RPE cells be derived from pluripotent stem cells (especially iPS cells).

In the present invention, the term "pluripotent stem cell" refers to a cell having self-renewal ability and pluripotency, a stem cell capable of being cultured *in vitro* and having an ability to differentiate into all of cell lineages belonging to three germ layers (ectoderm, mesoderm, endoderm) (pluripotency).

Examples of the pluripotent stem cell include an embryonic stem cell (ES cell) and an induced pluripotent stem cell (iPS cell). Pluripotent stem cells to be used in the present invention are mammalian pluripotent stem cells, preferably pluripotent stem cells of rodents, carnivores, or primates, more preferably human pluripotent stem cells. Examples of the mammal here include primates such as human and monkey, rodents such as mouse, rat, hamster, and guinea pig, carnivores such as canine and cat, and other swine, bovine, goat, horse, sheep, and rabbit.

Embryonic stem cells can be produced by, for example, culturing an inner cell mass present in the pre-implantation blastocyst stage embryo on a feeder cell or in a medium containing LIF. Specific production methods of the embryonic stem cells are described in, for example WO96/22362, WO02/101057, US5,843,780, US6,200,806, and US6,280,718. The embryonic stem cells are available from given organizations, or a commercially available product can be purchased. For example, human embryonic stem cells, KhES-1, KhES-2 and KhES-3, are available from Institute for Frontier Medical Sciences, Kyoto University. EB5 cell, a mouse embryonic stem cell, is available from RIKEN, and D3 cell line, a mouse embryonic stem cell, is available from ATCC. Note that the human embryonic stem cells are established from human embryos within 14 days of fertilization.

Nuclear transfer ES cell (ntES cell), one of the ES cells, can be established from a clone embryo produced by transplanting the nucleus of a somatic cell into an enucleated egg.

The term "induced pluripotent stem cell" refers to a cell induced to have pluripotency by reprogramming a somatic cell by a known method. Specific examples thereof include a cell induced to have pluripotency by reprogramming somatic cells such as fibroblasts, skin cells, and peripheral blood mononuclear cells by introduction of any combination of a plurality of reprogramming factors selected from the group consisting of genes such as Oct3/4, Sox2, Klf4, Myc (c-Myc, N-Myc, L-Myc), Glis1, Nanog, Sall4, lin28, and Esrrb. Examples of preferred combination of the reprogramming factors include (1) Oct3/4, Sox2, Klf4, and Myc (c-Myc or L-Myc), and (2) Oct3/4, Sox2, Klf4, Lin28 and L-Myc (Stem Cells, 2013; 31:458-466).

Induced pluripotent stem cells were first established by Yamanaka et al. in mouse cell in 2006 (Cell, 2006, 126(4), pp. 663-676) and also established in human fibroblast in 2007 (Cell, 2007, 131(5) pp. 861-872; Science, 2007, 318(5858) pp. 1917-1920; Nat. Biotechnol., 2008, 26(1) pp. 101-106). Various improvements have thereafter been made in an induction method of induced pluripotent stem cells, and a specific production method of, for example, a mouse induced pluripotent stem cell is described in Cell. 2006 Aug. 25; 126(4):663-76, and that of a human induced pluripotent stem cell is described in Cell. 2007 Nov. 30; 131(5):861-72.

Besides the production method based on direct reprogramming by gene expression, induced pluripotent stem cells can also be obtained from somatic cell by addition of a compound and the like (Science, 2013, 341, pp. 651-654).

It is also possible to obtain established induced pluripotent stem cells and, for example, human induced pluripotent cell lines established by Kyoto University such as 201B7 cells, 201B7-Ff cells, 253G1 cells, 253G4 cells, 1201C1 cells, 1205D1 cells, 1210B2 cells, and 1231A3 cells are available from Kyoto University and iPS Academia Japan, Inc. As the established induced pluripotent stem cells, for example, Ff-I01 cells, Ff-I14 cells, and QHJI01 cells established by Kyoto University are available from Kyoto University.

The somatic cells used in the production of induced pluripotent stem cells are not particularly limited, and specific examples thereof include fibroblasts and blood-lineage cells (e.g., peripheral blood mononuclear cells or T cells, cord blood-derived cells). Examples of the fibroblasts include those derived from dermis.

When induced pluripotent stem cells are produced through reprogramming by the expression of several reprogramming factors, the means for gene expression is not particularly limited, and a gene transfer method or a direct injection method of protein, which are well known to those skilled in the art, can be used. Specific examples of the gene transfer method include an infection method using a virus vector (e.g., retrovirus vector, lentivirus vector, Sendaivirus vector, adenovirus vector, adeno-associated virus vector), and a calcium phosphate method, lipofection method, RetroNectin method, or electroporation method, each using a plasmid vector (e.g., plasmid vector, episomal vector) or RNA vector.

An induced pluripotent stem cell can be produced in the presence of a feeder cell or absence of feeder cells (feeder-free). When an induced pluripotent stem cell is produced in the presence of a feeder cell, the induced pluripotent stem cell can be produced by a known method in the presence of an undifferentiated maintenance factor. Although a medium to be used for producing an induced pluripotent stem cell in the absence of feeder cells is not particularly limited, a known maintenance medium for embryonic stem cells and/or induced pluripotent stem cells, and a medium for establishing an induced pluripotent stem cell under feeder-free conditions can be used. Examples of the medium for establishing an induced pluripotent stem cell under feeder-free conditions include feeder-free media such as Essential 8 medium (E8 medium), Essential 6 medium, TeSR medium, mTeSR medium, mTeSR-E8 medium, Stabilized Essential 8 medium, and StemFit(R). In the production of an induced pluripotent stem cell, for example, the induced pluripotent stem cell can be produced by gene transfer of 4 factors of Oct3/4, Sox2, Klf4, and Myc into a somatic cell by using a Sendaivirus vector in the absence of feeder cells.

The pluripotent stem cells to be used in the present invention is preferably induced pluripotent stem cells of rodents, carnivores, or primates, more preferably human induced pluripotent stem cells.

Although those skilled in the art can perform maintenance culture or expansion culture of pluripotent stem cells by a well-known method, it is preferable that the pluripotent stem cells be subjected to maintenance culture or expansion culture under serum-free conditions and in the absence of feeder cells from the viewpoint of the safety of graft cell production, for example.

Genetically-modified pluripotent stem cells can be produced by using, for example, a homologous recombination technique. Examples of the gene on the chromosome to be modified include a cell marker gene, a histocompatibility antigen gene, a gene related to a disease based on a disorder of retinal pigment epithelial cells. A target gene on the chromosome can be modified using the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); and Biomanual Series 8, Gene Targeting, Making of Mutant Mouse using ES cell, YODOSHA CO., LTD. (1995).

Specifically, for example, the genomic DNA comprising the target gene to be modified (e.g., cell marker gene, histocompatibility antigen gene, disease-related gene) is isolated, and a targeting vector for homologous recombination of the target gene is produced using the isolated genomic DNA. After the produced targeting vector is introduced into stem cells, the cells in which homologous recombination has occurred between the target gene and the targeting vector are selected, whereby stem cells having the modified gene on the chromosome can be produced.

Examples of a method of isolating genomic DNA comprising the target gene include known methods described in Molecular Cloning, A Laboratory Manual, Second Edition, Cold Spring Harbor Laboratory Press (1989) and Current Protocols in Molecular Biology, John Wiley & Sons (1987-1997). The genomic DNS comprising the target gene can also be isolated using genomic DNA library screening system (manufactured by Genome Systems, Inc.) and Universal GenomeWalker Kits (manufactured by CLONTECH Laboratories, Inc.), for example. A polynucleotide encoding the target protein can also be used instead of genomic DNA. The polynucleotide can be obtained by amplifying the corresponding polynucleotide by the PCR method.

Production of targeting vector for homologous recombination of the target gene and efficient selection of a homologous recombinant can be performed according to the methods described in Gene Targeting, A Practical Approach, IRL Press at Oxford University Press (1993); Biomanual Series 8, Gene Targeting, Making of Mutant Mouse using ES cell, YODOSHA CO., LTD. (1995), for example. As the targeting vector, any of replacement type or insertion type can be used. As the selection method, methods such as positive selection, promoter selection, negative selection, and polyA selection can be used.

Examples of a method of selecting the desired homologous recombinant from the selected cell lines include Southern hybridization method and PCR method for the genomic DNA.

The method for producing a retinal pigment epithelial cell from a pluripotent stem cell is not particularly limited as long as it is a known method, and may be, for example, the methods described in WO2005/070011, WO2006/080952, WO2009/051671, WO2011/063005, WO2012/173207, WO2015/053375, WO2014/087244, WO2014/121077, WO2015/053376, WO2015/068505, WO2017/043605, Stem Cell Reports, 2(2), 205-218 (2014), and Cell Stem Cell, 10(6), 771-785 (2012). For example, the method may be a production method of a retinal pigment epithelial cell, including the following steps (1) and (2) disclosed in WO2017/043605:
(1) a first step of culturing a pluripotent stem cell in a medium containing an FGF receptor inhibitor and/or an MEK inhibitor for a period of not more than 30 days (preferably 2 days to 13 days or 2 days to 6 days, more preferably 4 days to 6 days), and
(2) a second step of culturing the cell obtained in the first step in the presence of a Nodal signal transduction pathway inhibitor and/or a Wnt signal transduction pathway inhibitor to form a retinal pigment epithelial cell.

The production method of a retinal pigment epithelial cell may also include the following steps (1) and (2):
(1) a first step of culturing a pluripotent stem cell in a medium containing an FGF receptor inhibitor and/or an MEK inhibitor for a period sufficient for inducing gene expression of at least one eye field transcription factor and of not more than 30 days, and
(2) a second step of culturing the cell obtained in the first step in the presence of a Nodal signal transduction pathway inhibitor and/or a Wnt signal transduction pathway inhibitor to form a retinal pigment epithelial cell.

The production method of a retinal pigment epithelial cell may further include the following steps (1) and (2):
(1) a first step of culturing a pluripotent stem cell in a medium containing an FGF receptor inhibitor and/or an MEK inhibitor for a period sufficient for inducing gene expression of at least one of PAX6, LHX2, and SIX3 and of not more than 30 days, and
(2) a second step of culturing the cell obtained in the first step in the presence of a Nodal signal transduction pathway inhibitor and/or a Wnt signal transduction pathway inhibitor to form a retinal pigment epithelial cell.

The FGF receptor inhibitor is not particularly limited as long as it is a substance capable of suppressing signal transduction mediated by FGF, and may be any of proteins, nucleic acids, and low-molecular-weight compounds. Herein, FGF forms a family consisting of at least 22 species. Example of the representative FGF receptor include FGFR1, FGFR2, FGFR3, and FGFR4, and the FGF receptor inhibitor is a substance that inhibits one, a plurality, or all of them. Examples of the substance include, but are not limited to, a substance that directly acts on FGF or FGF receptors (e.g., antibody, aptamer), a substance that suppresses expression of a gene encoding FGF or FGF receptors (e.g., antisense oligonucleotide, siRNA), a substance that inhibits binding of FGF receptors to FGF (e.g., soluble FGF receptor, FGF antagonist), and a substance that inhibits physiological activity caused by signal transduction by FGF receptors, such as a low-molecular-weight compound including PD173074 (N-[2-[[4-(diethylamino)butyl]amino]-6-(3,5-dimethoxyphenyl)pyrido[2,3-d]pyrimidin-7-yl]-N'-(1,1-dimethylethyl)urea) or SU5402 (2-[(1,2-dihydro-2-oxo-3H-indol-3-ylidene)methyl]-4-methyl-1H-pyrrole-3-propanoic acid) that inhibits tyrosine kinase activity of FGF receptors by ATP competition. Only one kind of the substance may be used, or two or more kinds thereof may be used in combination. Here, PD173074 and SU5402 are known FGF receptor inhibitors, and a commercially available product and the like can be obtained as appropriate. Preferred examples of the FGF receptor inhibitors include PD173074 and SU5402.

The MEK inhibitor is not particularly limited as long as it is a substance that inhibits expression or activity of MEK family, and may be any of proteins, nucleic acids, and low-molecular-weight compounds. Example of the representative MEK family include MEK1, MEK2, and MEK3, and the MEK inhibitor is a substance that inhibits the expression or activity of one, a plurality, or all of these MEK families. Examples of the substance include, but are not limited to, a substance that suppresses expression of a gene encoding various MEK (e.g., antisense oligonucleotide, siRNA), a substance that inhibits enzyme activity of various MEK, such as a low-molecular-weight compound including PD0325901 (N-[(2R)-2,3-dihydroxypropoxy]-3,4-difluoro-2-[(2-fluoro-4-iodophenyl)amino]-benzamide), PD184352 ((2-[(2-chloro-4-iodophenyl)amino]-N-cyclopropylmethoxy)-3,4-difluorobenzamide), PD98059 (2'-amino-3'-methoxyflavone), U0126(1,4-diamino-2,3-dicyano-1,4-bis[2-aminophenylthio]butadiene), MEK162 (5-[(4-bromo-2-fluorophenyl)amino] -4-fluoro-N-(2-hydroxyethoxy)-1-methyl-1H-benzimidazole-6-carboxamide), SL327(α-[amino[(4-aminophenyl)thio]methylene]-2-(trifluoromethyl)benzeneacetonitrile), TAK-733 ((R)-3-(2,3-dihydroxypropyl)-6-fluoro-5-(2-fluoro-4-iodophenylamino)-8-methylpyrido [2,3-d] pyrimidine-4,7 (3H,8H)-dione), or AZD-8330 (2-(2-fluoro-4-iodophenylamino)-N-(2-hydroxyethoxy)-1,5-dimethyl-6-oxo-1,6-dihydropyridine-3-carboxamide). One kind of the substance may be used, or two or more kinds thereof may be used in combination. Here, PD0325901, PD184352, PD98059, U0126, MEK162, SL327, TAK-733, and AZD-8330 are known MEK inhibitors, and a commercially available product and the like can be obtained as appropriate. Preferred examples of the MEK inhibitors include PD0325901, PD184352, U0126, TAK-733, and AZD-8330.

The Nodal signal transduction pathway inhibitor is not particularly limited as long as it can suppress signal transduction mediated by Nodal, and may be any of proteins, nucleic acids, and low-molecular-weight compounds. The signal mediated by Nodal is transduced via a Nodal receptor. The Nodal receptor is present as a heterodimer of TYPE I receptor (ALK (activin receptor-like kinase)-4, ALK-5, ALK-7) and TYPE II receptor (ActRII). Examples of the Nodal signal transduction pathway inhibitor include, but are not limited to, a substance that directly acts on Nodal or Nodal receptors (anti-Nodal antibody, anti-Nodal receptor antibody etc.), a substance that suppresses expression of a gene encoding Nodal or Nodal receptors (e.g., antisense oligonucleotide, siRNA etc.), a substance that inhibits binding of Nodal receptor to Nodal (Lefty-A, Lefty-B, Lefty-1, Lefty-2, soluble Nodal receptor etc.), and a substance that inhibits physiological activity caused by signal transduction by Nodal receptors, such as low-molecular-weight compounds including SB-431542 (SB431542) (4-[4-(1,3-benzodioxol-5-yl)-5-(pyridin-2-yl)-1H-imidazol-2-yl]benzamide) that inhibits kinase activity of TYPE I receptor by ATP competition. SB-431542 is a known Nodal signal inhibitor, and a commercially available product and the like can be obtained as appropriate. Preferred examples of the Nodal signal inhibitor include SB-431542.

The Wnt signal transduction pathway inhibitor is not particularly limited as long as it can suppress signal transduction mediated by Wnt, and may be any of proteins, nucleic acids, and low-molecular-weight compounds. The signal mediated by Wnt is transduced via a Wnt receptor present as a heterodimer of Frizzled (Fz) and LRP5/6 (low-density lipoprotein receptor-related protein 5/6). Examples of the Wnt signal transduction pathway inhibitor include, but are not limited to, a substance that directly acts on Wnt or Wnt receptors (anti-Wnt antibody, anti-Wnt receptor antibody etc.), a substance that suppresses expression of a gene encoding Wnt or Wnt receptors (e.g., antisense oligonucleotide, siRNA), a substance that inhibits binding of Wnt receptors to Wnt (soluble Wnt receptor, dominant negative Wnt receptor or the like, Wnt antagonist, Dkk1, Cerberus protein etc.), and a substance that inhibits physiological activity caused by signal transduction by Wnt receptor, such as low-molecular-weight compounds including casein kinase I inhibitors CKI-7 (N-(2-aminoethyl)-5-chloroisoquinoline-8-sulfonamide) and D4476 (4-[4-(2,3-dihydro-1,4-benzodioxin-6-yl)-5-(2-pyridinyl)-1H-imidazol-2-yl]benzamide), IWR-1-endo (IWR1e) (4-[(3aR,4S,7R,7aS)-1,3,3a,4,7,7a-hexahydro-1,3-dioxo-4,7-methano-2H-isoindol-2-yl]-N-8-quinolinyl-benzamide) that stabilizes β-catenin destruction complex by inhibiting metabolic turnover of Axin, and IWP-2 (N-(6-methyl-2-benzothiazolyl)-2-[(3,4,6,7-tetrahydro-4-oxo-3-phenylthieno[3,2-d]pyrimidin-2-yl)thio]acetamide) that inactivates Porcupine (Porcn) as a membrane-bound O-acyltransferase (MBOAT) and suppresses palmitoylation of Wnt protein. CKI-7, D4476, IWR-1-endo (IWR1e), IWP-2 and the like are known Wnt signal inhibitors, and a commercially available product and the like can be obtained as appropriate. Preferred examples of the Wnt signal inhibitor include CKI-7.

As used herein, the term "eye field transcription factor" refers to a gene expressed in the eye field region in an early developmental stage, and for example, ET(Tbx3), Rx1(Rax), Pax6, Six3, Lhx2, Tlx(Nr2e1), and Optx2(Six6) have been identified. These eye field transcription factors can be used as markers of early eye formation stage.

The number of days of the first step is not particularly limited as long as it is within the period when the cells generated by exposure of pluripotent stem cells to, for example, the FGF receptor inhibitor and/or MEK inhibitor, maintain differentiation potency into retinal pigment epithelial cells, and the pluripotent stem cells in the first step are cultured for a period of not more than 30 days. The period may vary according to the line of the pluripotent stem cells to be used and the like, but is generally 2 days or more, preferably 3 days or more, more preferably 4 days or more. In the first step, the pluripotent stem cells are still more preferably cultured for 2 days to 13 days or 2 days to 6 days, further preferably 4 days to 6 days.

A culture period of the second step is not particularly limited as long as it is a period capable of inducing retinal pigment epithelial cells of interest, but retinal pigment epithelial cells can be produced, as an example of such culture period, generally on days 14 to 40 calculating from the start of the second step. Those skilled in the art can confirm the generation of retinal pigment epithelial cells based on, for example, the expression of a cell marker (RPE65(retinal pigment epithelial cell), Mitf (retinal pigment epithelial cell), BEST1 (retinal pigment epithelial cell), CRALBP (retinal pigment epithelial cell) etc.), presence of melanin granules (brown-black), intercellular tight junction, and characteristic polygonal or cobblestone-like cell morphology, and determine the culture period by confirming them.

After the completion of the second step, it is preferable to exchange the medium with a maintenance medium for retinal pigment epithelial cells and further culture the same. For example, the cells are cultured for about 5 to 20 days while exchanging the total amount of the medium at a frequency of once or more in 3 days. As the maintenance medium for retinal pigment epithelial cells, for example, those described in IOVS, March 2004, Vol. 45, No. 3, Masatoshi Haruta, et. al., IOVS, November 2011, Vol. 52, No. 12, Okamoto and Takahashi, J. Cell Science 122 (17), Fumitaka Osakada, et. al., IOVS, February 2008, Vol. 49, No. 2, Gamm, et. al. can be used, which are composed of a basal medium, a serum, and/or a serum replacement, and other components.

The retinal pigment epithelial cells can also be produced by omitting the first step and conducting only the second step.

When retinal pigment epithelial cells are produced by adhesion culture, the retinal pigment epithelial cells can be adhered to each other to form a sheet-like structure. Therefore, a sheet of retinal pigment epithelial cells that can be transplanted into a patient can be produced. In addition, the sheet of retinal pigment epithelial cells (hereinafter sometimes referred to as "RPE sheet") can also be prepared as a suspension that can be transplanted into a patient by recovering the retinal pigment epithelial cells as independent single cells through dispersion by a method well known to those skilled in the art using an enzyme such as trypsin, and then suspending them in the transplantation medium of the present invention.

The term "adhesion culturing" refers to culturing under conditions in which a cell or cell aggregate is adhered to a culture vessel material or the like. In this case, adhesion of cells means that a strong cell-substratum junction is formed between a cell or cell aggregate and a culture vessel material. In other words, adhesion culturing refers to culturing under conditions in which a strong cell-substratum junction is formed between a cell or cell aggregate and a culture vessel material or the like. Those skilled in the art can appropriately set the culturing conditions such as a culture vessel material to conduct adhesion culturing.

The retinal pigment epithelial cells can also be produced by suspension culturing. In other words, an embryoid body (also referred to as EB) can be produced from pluripotent stem cells and induced to differentiate into retinal pigment epithelial cells to produce a cell aggregate containing retinal pigment epithelial cells. The cell aggregate can also be prepared as a suspension that can be transplanted into a patient by recovering the retinal pigment epithelial cells as independent single cells through dispersion by a method well known to those skilled in the art using an enzyme such as trypsin, and then suspending them in the transplantation medium.

### 2. Transplantation Medium

The transplantation medium of the present invention is a transplantation medium for subretinal transplantation of retinal pigment epithelial cells and may contain hyaluronic acid and a pharmaceutically acceptable aqueous liquid, in which the viscosity of the transplantation medium at a shear rate of 2 (1/s) at 25°C is 1 to 6 mPa·s, preferably 1.3 to 5.5 mPa·s, 1.5 to 4 mPa·s, more preferably 2 to 3 mPa·s. By using the predetermined viscosity range, it is possible to suppress cell leakage into the vitreous body and prolonged retinal detachment upon subretinal transplantation of retinal pigment epithelial cells, thereby reducing side effects such as epiretinal membrane and retinal detachment, as well as improving engraftment of retinal pigment epithelial cells at the transplantation site.

In the transplantation medium of the present invention, the viscosity at a shear rate of 1000 (1/s) at 25°C is preferably 3 mPa·s or less, more preferably 2 mPa·s or less, and the difference in viscosity between the viscosity at a shear rate of 1 (1/s) at 25°C and the viscosity at a shear rate of 10 (1/s) is 0.3 to 2.0 mPa·s, more preferably 0.4 to 1.8 mPa·s, 0.5 to 1.5 mPa·s, or 0.6 to 1.3 mPa·s. In one aspect, the viscosity at a shear rate of 1000 (1/s) at 25°C is 1 mPa·s or more, 1.1 mPa·s or more, or 1.2 mPa·s or more.

The transplantation medium of the present invention may contain chondroitin sulfate, but is preferably free of chondroitin sulfate. Both hyaluronic acid and chondroitin sulfate are mucopolysaccharides present in the body and function as thickening components of the transplantation medium.

In the present specification, hyaluronic acid is a linear polymeric polysaccharide with a molecular weight of tens of thousands to millions, the linear polymeric polysaccharide having a structure in which D-glucuronic acid and D-N-acetylglucosamine are linked alternately by β-1,4 and β-1,3 glycosidic bonds. Hyaluronic acid has high water-holding capacity and viscosity and is a substance widely used in, for example, pharmaceuticals and cosmetics. As used herein, the term "hyaluronic acid" refers to a concept including both free hyaluronic acid and a salt thereof. Examples of the salt of hyaluronic acid include alkali metal salts and alkaline earth metal salts of hyaluronic acid, and specifically, commercially available sodium hyaluronate, potassium hyaluronate, and the like can be used.

Chondroitin sulfate is mucopolysaccharide with a structure in which sulfuric acid is bound to a sugar chain composed of two repeating saccharides D-glucuronic acid (GlcA) and N-acetyl-D-galactosamine (GalNAc). As used herein, the term "chondroitin sulfate" refers to a concept including both free chondroitin sulfate and a salt thereof. Examples of the salt of chondroitin sulfate include alkali metal salts and alkaline earth metal salts of chondroitin sulfate, and specifically, commercially available sodium chondroitin sulfate, potassium chondroitin sulfate, and the like can be used.

As hyaluronic acid or a salt thereof, a commercially available aqueous solution of hyaluronic acid or a salt thereof can be used. Specific examples include VISCOAT(R) 0.5 ophthalmic viscoelastic substance containing 3% sodium hyaluronate, 4% chondroitin sulfate sodium, sodium dihydrogen phosphate monohydrate, sodium dihydrogen phosphate anhydrous, and a tonicity agent as components; PROVISC(R) 0.85 ophthalmic viscoelastic substance 1% containing 1% sodium hyaluronate, disodium hydrogen phosphate anhydrous, sodium dihydrogen phosphate monohydrate, pH adjusters (two components), and a tonicity agent as components; OPEGAN(R) 0.6 Ophthalmic Viscoelastic Preparation containing 0.6% sodium hyaluronate, sodium chloride, sodium dihydrogen phosphate, and sodium hydrogen phosphate as components; OPEGAN (R) 1.1 Ophthalmic Viscoelastic Preparation containing 1.1% sodium hyaluronate, sodium chloride, sodium dihydrogen phosphate, and sodium hydrogen phosphate as components; and Hyalein(R) Mini ophthalmic solution 0.3% containing 0.3% sodium hyaluronate, ε-aminocaproic acid, disodium edetate hydrate, potassium chloride, sodium chloride, and a pH-adjuster as components. The commercially available aqueous solution of hyaluronic acid or a salt thereof is preferably free of a substance that affects the viscosity (e.g., a thickener) other than hyaluronic acid and chondroitin sulfate, and is more preferably free of a substance that affects the viscosity (e.g., a thickener) other than hyaluronic acid.

The "pharmaceutically acceptable aqueous liquid" in the transplantation medium of the present invention is not particularly limited as long as it is an aqueous solution that can be injected into the body and has physical properties suitable for transplantation, and may be, for example, an aqueous solution containing a buffer, a transfusion, saline, injectable water, or perfusate. Preferred examples thereof include a buffer.

Examples of the "pharmaceutically acceptable aqueous liquid" include an aqueous solution containing a balanced salt (i.e., a balanced salt solution). In the aqueous solution containing a balanced salt, a buffer, a tonicity agent, a pH adjuster, an antioxidant, a chelating agent, or the like can be appropriately selected and contained within a range that does not affect the viability of retinal tissue to be transplanted.

Examples of the buffer include a phosphoric acid buffer, a boric acid buffer, a citrate buffer, a tartaric acid buffer, an acetate buffer, amino acid, and ε-aminocaproic acid.

Examples of the tonicity agent include saccharides such as sorbitol, glucose, and mannitol, polyhydric alcohols such as glycerol and propylene glycol, salts such as sodium chloride, and boric acid.

Examples of the chelating agent include sodium edetate and citric acid.

Examples of the pH adjuster include sodium hydroxide, potassium hydroxide, sodium carbonate, sodium hydrogen carbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), and tartaric acid or a salt thereof (sodium tartrate etc.).

Examples of the antioxidant include ascorbic acid, glutathione, sodium hydrogen sulfite, dry sodium sulfite, sodium pyrosulfite, and tocopherol.

Specific examples of the "pharmaceutically acceptable aqueous liquid" include an aqueous solution containing one or a plurality of components selected from saccharides such as glucose, inorganic salts such as calcium chloride, sodium chloride, magnesium chloride, potassium chloride, and magnesium sulfate, inorganic materials such as sodium hydrogen carbonate, sodium acetate, sodium citrate, sodium dihydrogen phosphate, sodium hydrogen phosphate, disodium hydrogen phosphate anhydrous, and hydrochloric acid, and chelating agents such as edetate (e.g., sodium edetate).

To prepare the pharmaceutically acceptable aqueous liquid, a commercially available liquid can be used as an intraocular irrigating/washing solution. Specific examples of intraocular irrigating/washing solution that can be used include an aqueous solution containing 1.5 mg of glucose, 6.6 mg of sodium chloride, 0.36 mg of potassium chloride, 0.18 mg of calcium chloride hydrate, 0.3 mg of magnesium sulfate hydrate, and 2.1 mg of sodium hydrogen carbonate in 1 mL, and sodium citrate hydrate, sodium acetate hydrate, and hydrochloric acid as additives, which is commercially available as OPEGUARD(R)-MA intraocular irrigating solution (Senju Pharmaceutical Co., Ltd.); and an oxiglutatione ocular irrigating solution. In addition, Hank's balanced salt solution (HBSS), Eagle's balanced salt solution (EBSS), phosphate-buffered saline (PBS), Dulbecco's phosphate-buffered saline (DPBS), and the like can also be used to prepare a pharmaceutically acceptable aqueous liquid.

In one aspect of the transplantation medium of the present invention, examples thereof include a transplantation medium containing only hyaluronic acid as a thickening component, and specifically, ophthalmic viscoelastic substance PROVISC(R) 0.85 ophthalmic viscoelastic substance 1% (Alcon Japan Ltd) containing 10 mg/mL of sodium hyaluronate without chondroitin sulfate can be prepared by 20 to 200-fold dilution (preferably 50 to 150-fold, approximately 100-fold dilution) with a balanced salt solution such as HBSS, for example. VISCOAT(R) 0.5 ophthalmic viscoelastic substance can be prepared by 20 to 200-fold solution (preferably 50 to 150-fold, approximately 100-fold dilution) with a balanced salt solution such as HBSS.

In one aspect of the transplantation medium of the present invention, VISCOAT(R) 0.5 ophthalmic viscoelastic substance (Alcon Japan Ltd) containing 30 mg/mL of sodium hyaluronate and 40 mg/mL of chondroitin sulfate sodium can be prepared by 20 to 200-fold solution (preferably 50 to 150-fold, approximately 100-fold dilution) with a balanced salt solution such as HBSS.

In one aspect of the transplantation medium of the present invention, OPEGAN(R) 0.6 ophthalmic viscoelastic substance can be prepared by 20 to 200-fold dilution with an aqueous liquid such as OPEGUARD. In another aspect, Hyalein(R) Mini ophthalmic solution 0.3% can be prepared by 6 to 60-fold dilution with an aqueous liquid such as OPEGUARD.

In one aspect of the transplantation medium of the present invention, examples thereof include a transplantation medium containing hyaluronic acid as a thickening component, and may further contain chondroitin sulfate, where the concentration of hyaluronic acid and chondroitin sulfate is not particularly limited as long as the viscosity of the transplantation medium at a shear rate of 2 (1/s) at 25°C can be maintained at 1.5 to 4 mPa·s, preferably 2 to 3 mPa·s. One aspect of the transplantation medium of the present invention includes a transplantation medium containing 0.005 w/v% to 0.15 w/v% (e.g., 0.007 w/v% to 0.04 w/v%, 0.008 w/v% to 0.035 w/v%, 0.009 w/v% to 0.03 w/v%, approximately 0.01 w/v%) of hyaluronic acid with an average molecular weight of 500,000 to 3.9 million (preferably 1.5 million to 3.9 million). Additional examples thereof includes a transplantation medium containing more than 0 w/v% (e.g., 0.001 w/v%) to 0.20 w/v% (or 0.1 w/v%, 0.05 w/v%) of chondroitin sulfate or a salt thereof with a molecular weight of 20,000 to 24,000. As described above, the transplantation medium of the present invention is more preferably free of chondroitin sulfate.

The transplantation medium of the present invention is preferably free of any viscosity-affecting substance other than hyaluronic acid and chondroitin sulfate (e.g., a thickener (thickening component)), or any viscosity-affecting substance other than hyaluronic acid (e.g., a thickener (thickening component)). Examples of the thickener include polysaccharides such as gum arabic, gumkaraya, xanthan gum, caseins, agar, alginate, α-cyclodextrin, dextrin, dextran, carrageenan, gelatin, collagen, pectin, starch, chitin and derivatives thereof, chitosan and derivatives thereof, elastin, heparin, heparinoid, heparin sulfate, heparan sulfate, hyaluronic acid, methylcellulose, and hydroxyethyl cellulose or derivatives thereof, ceramide, macrogol, glycerin, povidone, polyvinyl methacrylate, polyvinyl alcohol, polyacrylic acid, carboxyvinyl polymer, and polyethyleneimine.

The transplantation medium of the present invention is preferably free of an antibacterial agent and a preservative.

In this context, examples of the physical properties suitable for transplantation include pH and osmotic pressure. The pH of the transplantation medium is not particularly limited as long as it is in a neutral range, and the transplantation medium of the present invention may be adjusted to pH 6.5 to 8.0, preferably about pH 7.0 to 7.5. Examples of the osmotic pressure of the transplantation medium include hypotonic, isotonic, and hypertonic pressures, and it is preferable to be close to isotonic pressure. The osmotic pressure ratio may be adjusted to 0.7 to 1.3, preferably 0.9 to 1.1.

The transplantation medium of the present invention can be preferably subjected to sterilization treatment such as filter sterilization using a membrane filter.

### 3. Transplantation Composition

The transplantation composition of the present invention contains retinal pigment epithelial (RPE) cells for transplantation and the transplantation medium of the present invention. The transplantation composition of the present invention may be a cell suspension obtained by suspending retinal pigment epithelial cells for transplantation in a transplantation medium. The transplantation medium of the present invention is as described in 2. Transplantation Medium above. As retinal pigment epithelial cells for transplantation, retinal pigment epithelial cells described in 1. Definition can be used, preferably retinal pigment epithelial cells derived from human iPS cells.

The state of the retinal pigment epithelial cells is not particularly limited as long as the cells can be transplanted. In one aspect, the cells may be a sheet of retinal pigment epithelial cells that can be transplanted into a patient, dispersed retinal pigment epithelial cells, or a combination thereof. The retinal pigment epithelial cells may be sheet-free, dispersed retinal pigment epithelial cells.

It is preferable that in the transplantation composition, the retinal pigment epithelial cells for transplantation are contained at a concentration of 1 × 10⁶ cells/mL to 5 × 10⁷ cells/mL, and it is more preferably that the concentration is 2.5 × 10⁶ cells/mL to 1.5 × 10⁷ cells/mL or 5 × 10⁶ cells/mL to 1 × 10⁷ cells/mL. For single transplantation, it is preferable that the volume (dose volume) of the transplantation composition be 10 µL to 100 µL, and more preferable that the volume is, for example, 20 µL to 80 µL, 10 µL to 70 µL, 20 µL to 50 µL, or 30 µ L to 50 µL. For example, the dose may be 0.2 × 10⁵ cells to 5 × 10⁵ cells, or 1 × 10⁵ cells to 5 × 10⁵ cells. The transplantation composition of the present invention may be a cell suspension containing retinal pigment epithelial cells for transplantation at the above concentration.

The transplantation composition of the present invention is a composition for transplanting (or injecting) RPE cells into the subretinal space of the eyeball. The subject to which the transplantation composition of the present invention is injected may be a mammal (e.g., human, canine, cat, mouse, or rat, preferably human) having a disease based on a disorder of retinal pigment epithelial cells or a lesion of retinal pigment epithelial cells, for example, a mammal (e.g., a human, mouse, or rat, preferably human) having a retinal disease/retinal lesion such as macular degeneration (e.g., atrophic and exudative age-related macular degeneration), hereditary retinal disease, degeneration of retinal pigment epithelium (including retinitis pigmentosa and crystallin retinopathy), deletion defect/tear of retinal pigment epithelium, macular dystrophy (including Stargardt disease), cone dystrophy, rod dystrophy, or cone-rod dystrophy, and it is particularly preferable that the subject be a human having macular degeneration, degeneration/deletion, defect/tear of retinal pigment epithelium, or the like.

When the transplantation composition of the present invention is used for the treatment of retinitis pigmentosa, it may be used, for example, for the treatment of corrected visual acuity of less than 0.7, preferably less than 0.2.

When the transplantation composition of the present invention is used for the treatment of retinitis pigmentosa, examples of the disease includes diseases in which there is visual field narrowing, for example, diseases in which a visual field with a center of 20 degrees remains in the static visual field examination (which can be examined by Humphrey visual field examination), preferably a disease in which a visual field with a center of 10 degrees remains in the static visual field examination (which can be examined by Humphrey visual field examination), and more preferably a disease in which a visual field with a center of 5 degrees remains in the static visual field examination (which can be examined by Humphrey visual field examination).

When the transplantation composition of the present invention is used for the treatment of retinitis pigmentosa, it may be used, for example, for the treatment in which the MD value is less than - 30 dB in the Humphrey visual field examination (10-2).

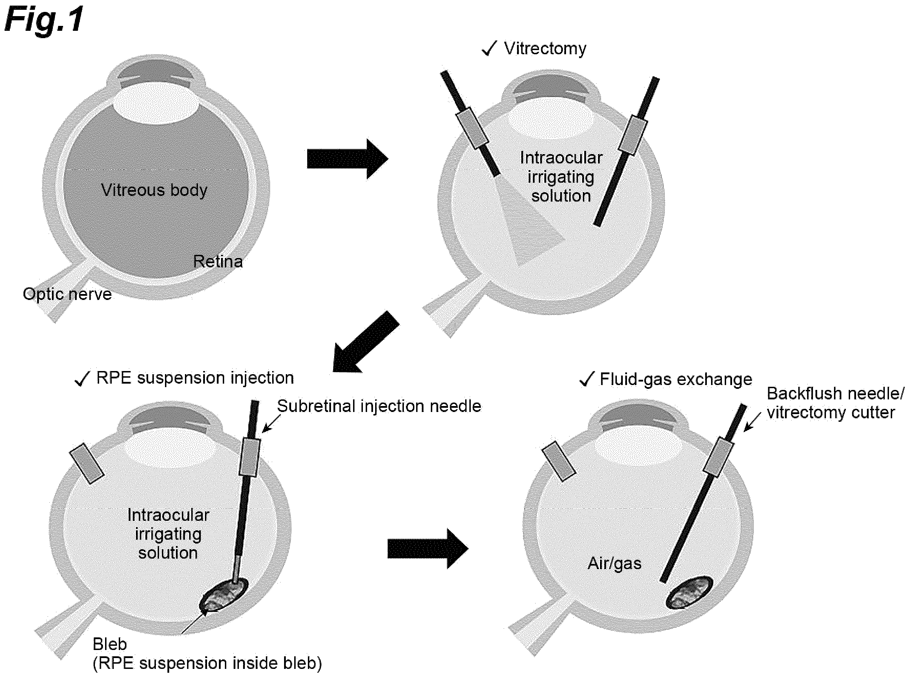

The subretinal transplantation (injection) method is not particularly limited as long as it is a known method, and examples thereof include injection using a syringe of an appropriate size or a transplantation device comprising a needle or a resin part, more specifically, the transvitreal cavity method and the transscleral approach. The outline of the transvitreal cavity method is shown in Figure 1, and as described in Example 1, it is performed by using a cataract and vitreous body surgical equipment to conduct a vitrectomy including the removal of the posterior vitreous membrane, and then administering an appropriate amount of the transplantation composition by injection into the subretinal space (between the sensory retina and retinal pigment epithelial cells) to form a bleb (hydraulically debrided retinal detachment). Thereafter, the vitreous cavity may be displaced with air if necessary. Besides the method of forming a bleb through injection of the transplantation composition itself, the method of forming a bleb includes a method of injecting the transplantation composition after first injecting an ocular irrigating solution (such as HBSS) to form a bleb and, if necessary, aspirating the liquid in the bleb.

The transscleral approach is a method of approaching from the outside of the eyeball, in which a portion of the sclera is incised and detached, a catheter is inserted into the choroidal space therefrom, and an appropriate amount of the transplantation composition can be administered by subretinal injection.

In the transvitreal cavity method, a few ports are made on the sclera for the insertion of surgical instruments. Since the transplantation composition of the present invention can be administered (inj ected) using a needle, a syringe, or the like, the method can be performed in a minimally invasive manner among transvitreal cavity methods. When the transplantation composition of the present invention is administered by the transvitreal cavity method, the transplantation composition of the present invention may be administered through the ports on the sclera to the affected area while forming a bleb.

The injection needle is not particularly limited, and examples thereof includes a metallic bevel needle and a polytip needle. A polytip needle is particularly preferred. In one aspect, the outer diameter of the injection needle may be 38 gauge, 39 gauge, or 40 gauge.

In the transvitreal cavity method, the transplantation composition or the retinal pigment epithelial cells for transplantation may leak from the bleb into the vitreous body, and the leaked retinal pigment epithelial cells may form the epiretinal membrane. On the other hand, in the transscleral approach, the transplantation composition rarely leaks into the vitreous body unless a hole is made on the retinal side. However, the epiretinal membrane may be formed by retinal pigment epithelial cells leaking from the bleb, even in the transscleral approach, because the needle may perforate the retinal side upon injection of a liquid into a narrow area. In addition, both the transvitreal cavity method and the transscleral approach cause retinal detachment. The formation of the epiretinal membrane and the prolonged retinal detachment are considered as adverse effects of subretinal injection. The transplantation medium in the transplantation composition of the present invention can suppress cell leakage into the vitreous body and prolonged retinal detachment by having a specific viscosity.

It is preferable that the transplantation method (injection method) for the transplantation medium or transplantation composition of the present invention be the transvitreal cavity method, and it is preferable to replace the vitreous body with air after the subretinal transplantation by the transvitreal cavity method.

One aspect of the present invention includes a method of treating a disease based on a disorder of retinal pigment epithelial cells or a damaged state of retinal pigment epithelial cells, the method including transplanting the transplantation composition of the present invention into a subject in need of transplantation (e.g., subretinal space of the eye suffering from an ophthalmologic disease). The transplantation composition of the present invention can be used as a therapeutic agent for a disease based on a disorder of retinal pigment epithelial cells, or in a state of damage to retinal pigment epithelial cells, to replenish the site of the damage. The diseases based on a disorder of retinal pigment epithelial cells or state of damage to retinal pigment epithelial cells can be treated by transplanting the transplantation composition of the present invention to a patient in need of transplantation with a disease based on a disorder of retinal pigment epithelial cells or in a state of damage to retinal pigment epithelial cells, and then replenishing the retinal pigment epithelial cells or the damaged retinal pigment epithelial cells.

### 4. Use, Therapeutic Agent, and Treatment Method

One aspect of the present invention provides use of the transplantation medium of the present invention, for subretinal transplantation of retinal pigment epithelial cells, containing hyaluronic acid and a pharmaceutically acceptable aqueous liquid, in which the viscosity of the transplantation medium at a shear rate of 2 (1/s) at 25°C is 1.5 to 4 mPa·s. One aspect of the present invention provides the transplantation medium for use in subretinal transplantation of retinal pigment epithelial cells.

One aspect of the present invention provides a therapeutic agent for a disease based on a disorder of retinal pigment epithelial cells, the therapeutic agent containing of the transplantation composition of the present invention. The disorder of retinal pigment epithelial cells and dose are as described above.

The therapeutic agent of the present invention may be injected in combination with an immunosuppressive agent. Those skilled in the art can select the immunosuppressive agent for use in transplantation.

The therapeutic agent of the present invention may be a cell suspension prepared in a medical institution using a transplantation medium and retinal pigment epithelial cells. In this case, the retinal pigment epithelial cells may be frozen and transported to the medical institution as a frozen preparation containing the retinal pigment epithelial cells. It is preferable that the retinal pigment epithelial cells be frozen in a dispersed state. The method of cryopreservation of retinal pigment epithelial cells is not limited as long as it is a method generally known for cryopreserving cells. For example, the cells can be frozen at -80°C and then cryopreserved with liquid nitrogen by using a commercially available cell cryopreservation solution StemCellBanker (Zenoaq). In a medical institution, the frozen preparation may be thawed at the time of use and suspended in a transplantation medium or the like for use in transplantation medicine. After thawing of the frozen preparation, the cells may be washed with a buffer solution such as a transplantation medium before suspension in the transplantation medium or the like.

One aspect of the present invention provides a method for treating a disease based on a disorder of retinal pigment epithelial cells, the method comprising a step of administering to a patient in need thereof an effective amount of the transplantation composition of the present invention or an effective amount of the therapeutic agent of the present invention.

The method of administering the transplantation composition or therapeutic agent of the present invention is not particularly limited as long as it is a known method, and examples thereof include the transvitreal cavity method and the transscleral approach described above, with the transvitreal cavity method being preferred.

Human subjects receive local (sub-Tenon's anaesthesia/ retrobulbar anaesthesia) or general anesthesia. When the transplantation composition or therapeutic agent is to be injected by the transvitreal cavity method, vitrectomy is done using an appropriate vitreous body surgical equipment, (e.g., CONSTELLATION Vision System). At this time, if necessary, triamcinolone acetonide (0.5 to 4 mg) may be injected into the vitreous body for the purpose of vitreous visualization. The transplantation composition or therapeutic agent may be injected into the affected area through a port on the sclera to form a bleb. If the injection method involves blebbing, it may further includes washing the vitreous cavity and air-exchanging the vitreous cavity of the human subject after injection.

In the treatment method of the present invention, an immunosuppressive agent may be administered in combination.

It is preferable to rest in a supine position for at least 12 hours after implementation of the treatment method of the present invention.

As an effect of injection (transplantation) of the transplantation composition or therapeutic agent or implementation of the treatment method of the present invention, retinal pigment epithelial cells are engrafted at the injection site of the subject (e.g., human), whereby the adhesion structure with the photoreceptor layer is reconstructed. As a result, the transplanted retinal pigment epithelial cells function to restore visual function or prevent further deterioration and maintain visual function as compared with conditions prior to injection of the therapeutic agent or implementation of the treatment method.

The above therapeutic effect can be confirmed by the above-described ophthalmologic examination. It can also be assessed with scales that measure health-related quality of life (QOL). These scales comprehensively assess the health condition from physical, mental, and social aspects, and there are also indices dedicated to visual function. Examples thereof include the Visual Function Questionnaire (VFQ-25), the Euro-QOL 5 Dimension-5 Level (EQ-5D-5L), the Health Utilities Index Mark 3 (HUI3).

### Examples

### <Example 1: Study of Influence on Cell Leakage Using Enucleated Porcine Eye>

The influence of the viscosity of the transplantation medium and postoperative air exchange in the vitreous cavity on cell leakage was examined *ex-vivo* using porcine eyes having an eyeball size close to that of a human. Retinal pigment epithelial (RPE) cells derived from human iPS cells (QHJI01s04 line) produced in the same manner as the method of Kuroda et al. (Stem Cell Res. 2019; 39:101514.) were used in all Examples. Porcine eyes extracted from slaughter pigs were obtained on the same day, and when the fundus was difficult to observe due to lens opacity, the lens was removed if necessary. A suspension of human iPS cell-derived RPE cells were subretinally injected (transplanted) by the method described below.

As shown in the schematic view of Figure 1, subretinal injection of the RPE cell suspension was conducted according to a general procedure of vitreous surgery (transvitreal cavity method). Using a cataract and vitreous body surgical equipment (CONSTELLATION Vision System LTX, manufactured by Alcon Japan Ltd), 0.5 to 4 mg of triamcinolone acetonide (manufactured by FUJIFILM Wako Pure Chemical Corporation, 209-10961) was injected intravitreally to visualize the vitreous body, and posterior vitreous detachment was created from the macula to perform peripheral vitrectomy. An ophthalmic surgical syringe and needle adapter was connected to the vitreous body surgical equipment using an appropriate tube and filled with RPE cell suspension. A subretinal injection needle (MedOne Cannula: Tapered PolyTip Cannula 25G/38G, 3219, MedOne Surgical, Inc./NIHON SURGE Inc.) was connected after removal of air at the tip using the ejection function of the vitreous body surgical equipment. The subretinal injection needle was inserted through the port, and the RPE cell suspension was subretinally administered by injection in an appropriate volume. Subretinal injection of the RPE cell suspension resulted in bleb formation. After injection, the vitreous cavity was thoroughly cleaned and, if necessary, exchanged with air.

The injected RPE cell suspension was obtained by suspending RPE cells labeled with the fluorescence labeling reagent PKH26 red fluorescent cell linker Mini kit (manufactured by Sigma-Aldrich, MINI26) in two types of transplant media at a concentration of 5.7 × 10⁷ cells/mL (air exchange condition) or 6.0 × 10⁶ cells/mL (no air exchange).

Viscous medium: Medium (7-fold dilution) obtained by mixing VISCOAT 0.5 ophthalmic viscoelastic substance (manufactured by Alcon Japan Ltd) and Hank's balanced salt solution HBSS(+)(manufactured by Thermo Fisher Scientific, Inc., 14025092) at a ratio of 1:6.

### Buffer only medium: HBSS(+) only medium

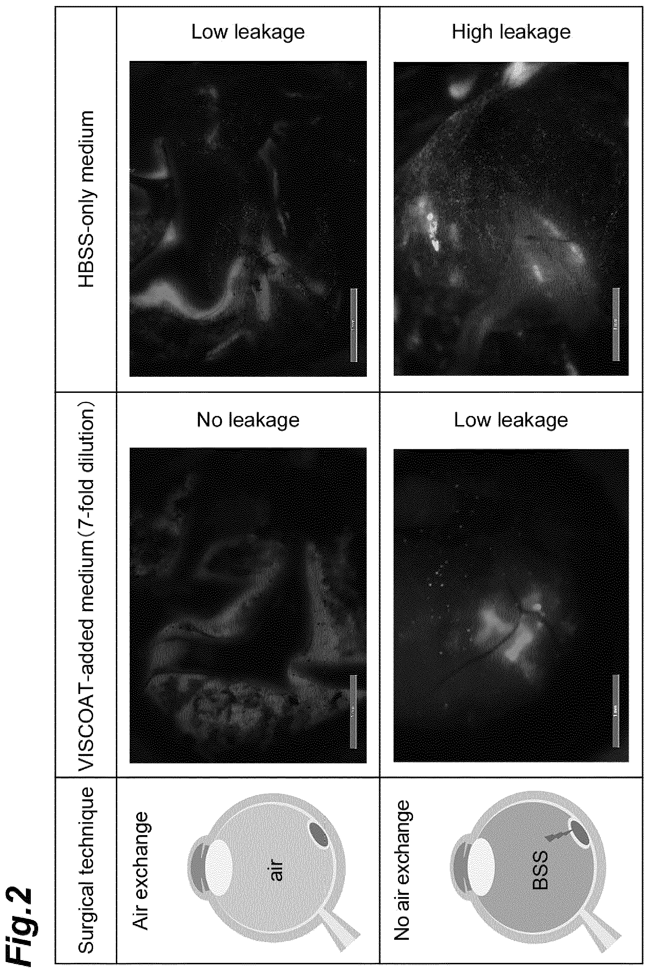

After injection, the vitreous cavity was allowed to stand for several hours, the hemisphare on the corneal side was removed to perform fundus fluorescence observation under a stereo fluorescence microscope (Leica M165FC, manufactured by Leica Microsystems), and the results are shown in Figure 2. It was found from Figure 2 that cell leakage was reduced by adding hyaluronic acid to the medium. In addition, no cell leakage was observed with the combined use of vitreous air exchange.

In addition, subretinal injection was carried out by varying the dilution factor of VISCOAT in the medium in the range of 7 to 21 times. It was found that the higher concentration of VISCOAT (lower dilution factor) resulted in higher resistance during injection, making it difficult to form the bleb. It was also confirmed that approximately 100 µL of the injection solution could be administered, but reflux from the injection site was observed when the dose was increased. It was confirmed that the RPE cells up to a concentration of 5.7 × 10⁷ cells/mL could be successfully injected without any problem.

### <Example 2: Transplantation Experiment in Cynomolgus Monkey>

To reduce cell leakage and improve subretinal cell engraftment, a dosing regimen was examined using cynomolgus monkeys whose ocular anatomy is very close to that of humans. Human iPS cell-derived RPE cells suspended in HBSS (+) (same as above) only or in a transplantation medium prepared by diluting the ophthalmic viscoelastic substance such as PROVISC(R) 0.85 ophthalmic viscoelastic substance 1% (Alcon Japan Ltd) (hereinafter referred to as PROVISC) or VISCOAT(R) 0.5 ophthalmic viscoelastic substance (Alcon Japan Ltd) (hereinafter referred to as VISCOAT) by 20-, 100-, or 200-fold dilution with HBSS (+) were subretinally injected to cynomolgus monkeys to examine the appropriate composition of the transplantation medium. In addition, the effect of the presence or absence of postoperative air exchange of the vitreous cavity was also examined. Herein, VISCOAT contains 30 mg/mL of sodium hyaluronate and 40 mg/mL of chondroitin sulfate sodium, and PROVISC contains 10 mg/mL of sodium hyaluronate without chondroitin sulfate.

Cambodian native cynomolgus monkeys (2.0 to 5.0 kg at the onset of habituation) were used in the study. One to two drops of tropicamide/phenylephrine hydrochloride ophthalmic solution (Mydrin(R)-P ophthalmic solution, Santen Pharmaceutical Co., Ltd.) and phenylephrine hydrochloride ophthalmic solution (Neosynesin Kowa 5% ophthalmic solution, Kowa Company, Ltd.) were each applied to the right eyes, to dilate the pupils. 10 mg/kg of Ketamine Hydrochloride (Ketalar for intramuscular injection 500 mg, Daiichi Sankyo Propharma Co., Ltd.) and 0.08 mg/kg of medetomidine hydrochloride (Domitor(R), Orion Corporation) were administered intramuscularly, followed by endotracheal intubation and controlled breathing using a mixed gas of oxygen and 0.5% to 2.0% isoflurane (ISOFLURANE Inhalation Solution "Pfizer", Mylan Inc.). The animals were fixed on the operating table in the supine position, and an appropriate amount (50 to 100 µL) of RPE cell suspension (2.5 × 10⁶ to 1.5 × 10⁷ cells/mL) was subretinally injected in the same manner as in Example 1. After injection, any leaked suspension was aspirated. In addition, the vitreous cavity was replaced with air as in Example 1, if necessary. The supine position was maintained under inhalation anesthesia for up to 3 hours to promote cell sedimentation and engraftment. To suppress rejection, immunosuppression was conducted using a once-daily intramuscular injection of cyclosporine (Sandimmun(R) for i.v. infusion 250mg, Novartis Pharma K.K.) and sub-Tenon's injection of triamcinolone acetonide (MaQaid(R) OPHTHALMIC INJECTION 40mg, WAKAMOTO PHARMACEUTICAL CO., LTD.) at surgery. After injection, the injected eyes were observed over time using a fundus camera (Kowa Optimized, VX-10α) and an OCT device (Heidelberg Engineering, SPECTRALIS OCT2).

The results of subretinal injection in cynomolgus monkeys are shown. When a suspension at a cell concentration of 1.5 × 10⁷ cells/mL was used, visibility of the injection needle tip was reduced at the time of injection in all media, while injection was possible without any problem at a cell concentration of 1.0 × 10⁷ cells/mL or less. Regarding the administration volume, it was confirmed that subretinal injection to one eye was possible in a range of 50 to 100 µL, and stable injection was possible with good reproducibility up to an dose volume of 70 to 80 µL.

The results of OCT findings up to 4 weeks later are shown in Table 1 and Table 2. From Table 1 and Table 2, the formation of epiretinal membrane (ERM) was observed in 7 out of 8 animals in which the vitreous cavity was not replaced with air and in 8 out of 23 animals in which the vitreous cavity was replaced with air, indicating the possibility that the leakage of the cell suspension could be suppressed and the formation of epiretinal membrane could be reduced by fluid air exchange.

In the group of transplantation media with 20-fold dilution of PROVISC or VISCOAT, prolonged retinal detachment around the injection site was observed in 8 out of 10 cases, while in the other groups, re-attachment of the retina at the injection site was confirmed by approximately 4 weeks after injection, except for one case of 200-fold diluted PROVISC. The medium with 20-fold dilution of PROVISC or VISCOAT did not have the effect of suppressing the formation of the epiretinal membrane. High viscosity reduced the leakage of suspensions, while prolonged retinal detachment left subretinal fluid for a long period of time, which might not have affected the sum of cells leaking to the vitreous side. In addition, ERM was observed more frequently in the transplantation medium with 200-fold dilution of PROVISC or VISCOAT. Although the reason is not clear, it is considered that leakage occurs at a viscosity equal to or less than a certain threshold value, and when the leaked hyaluronic acid is present in the vitreous cavity, cells are likely to form the ERM using the hyaluronic acid as a scaffold. On the other hand, the formation of ERM was not observed when a transplantation medium with 100-fold dilution of PROVISC or VISCOAT was used. Therefore, it was found that addition of 100-fold diluted PROVISC or VISCOAT to the medium is preferred for inhibition of cell leakage.

### [Table 1]

**Table 1. Prolonged Retinal Detachment/Formation of Epiretinal Membrane**

| PROVISC/VISCOAT | Prolonged retinal detachment | Epiretinal membrane without air exchange | Epiretinal membrane with air exchange |
|---|---|---|---|
| 20-fold dilution | 8/10 | 4/5 | 2/5 |
| 100-fold dilution | 0/3 | - | 0/3 |
| 200-fold dilution | 1/4 | - | 3/4 |
| No addition | 0/14 | 3/3 | 3/11 |

### [Table 2]

**Table 2. Prolonged Retinal Detachment/Formation of Epiretinal Membrane**

| Dilution factor | Ophthalmic viscoelastic substance | Prolonged retinal detachment | Epiretinal membrane without air exchange | Epiretinal membrane with air exchange |
|---|---|---|---|---|
| 20-fold dilution | PROVISC | 3/4 | 1/2 | 1/2 |
| | VISCOAT | 5/6 | 3/3 | 1/3 |
| 100-fold dilution | PROVISC | 0/2 | - | 0/2 |
| | VISCOAT | 0/1 | - | 0/1 |
| 200-fold dilution | PROVISC | 0/2 | - | 1/2 |
| | VISCOAT | 1/2 | - | 2/2 |
| - | HBSS(+) only | 0/14 | 3/3 | 3/11 |

At the end of the observation period (4, 13, and 26 weeks after surgery), the eyeballs were enucleated, fixed, and embedded in paraffin. Ten sections (3 to 4 µm thick) were prepared at 0.56 mm intervals to cover the blebs, and in order to evaluate engraftment, fluorescent immunostaining was performed according to a conventional method using one of the RPE cell markers, anti-human PMEL17 antibody (Novus Biologicals, NBP2-47780AF647, mouse or Abcam, ab137062, or rabbit), and anti-human CD147 antibody (R&D Systems, AF972, goat) as human cells. The stained specimens were observed with a fluorescence microscope (KEYENCE, BZ-X810) or a slide scanner (Zeiss, Axio Scan. Z1), and the regions including the positive sites were then imaged.

Although the anti-CD147 antibodies used showed a strong positive signal in a part of the region where human iPS cell-derived RPE cells were supposed to be engrafted, it was suggested that the anti-CD 147 antibodies also cross-react with monkey antigens since the entire retinal pigment epithelium layer of cynomolgus monkey was stained. Therefore, the anti-PMEL17 antibody-positive signal was used as a major indicator of engraftment and distribution of human iPS cell-derived RPE cells, and the anti-CD147 antibodies were used by multiple staining to facilitate the identification of the monkey retinal pigment epithelial layer during observation.

To quantitatively analyze the engraftment performance, the total score (0 to 30) in each individual was calculated by summing the results of 10 slices evaluated on the following four-level grading (Grades 0 to 3) for each slice according to the total number of engraftment sites with consecutive anti-PMEL17 antibody positivity (sites with consecutive subretinal positive cells observed over 100 µm or more).

### Four-Level Grading

Grade 0: no positive cells
Grade 1: minimal (no consecutive engraftment but positive cells were observed)
Grade 2: moderate (one or two consecutive engraftment sites were observed)
Grade 3: significant (three or more consecutive engraftment sites were observed)

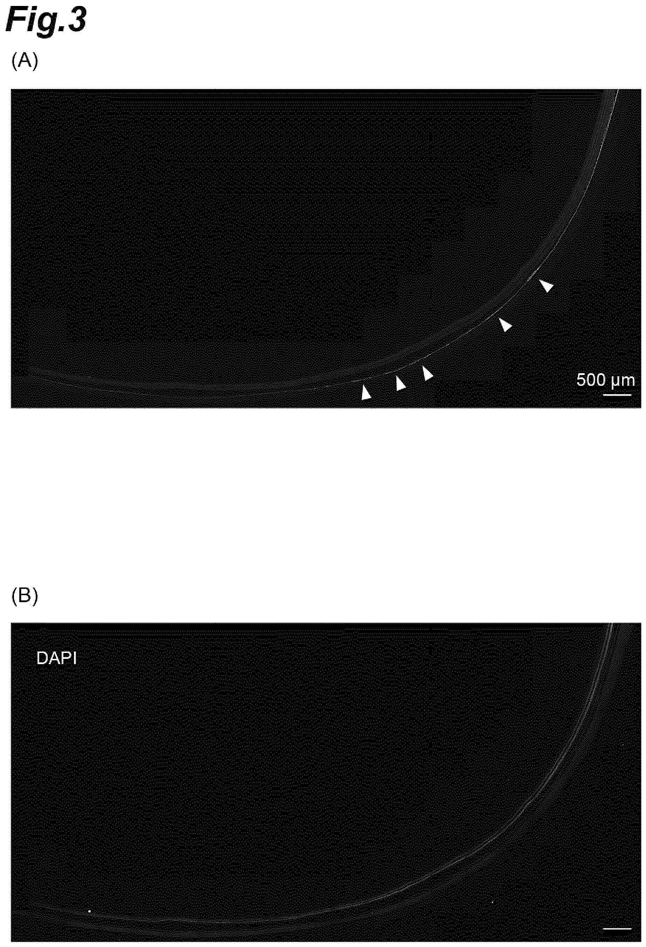

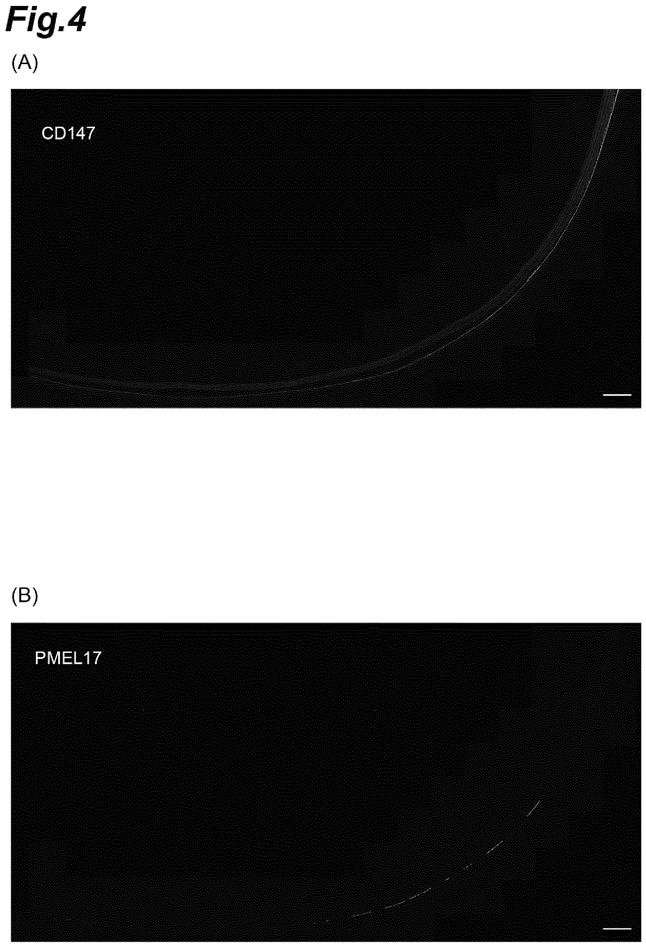

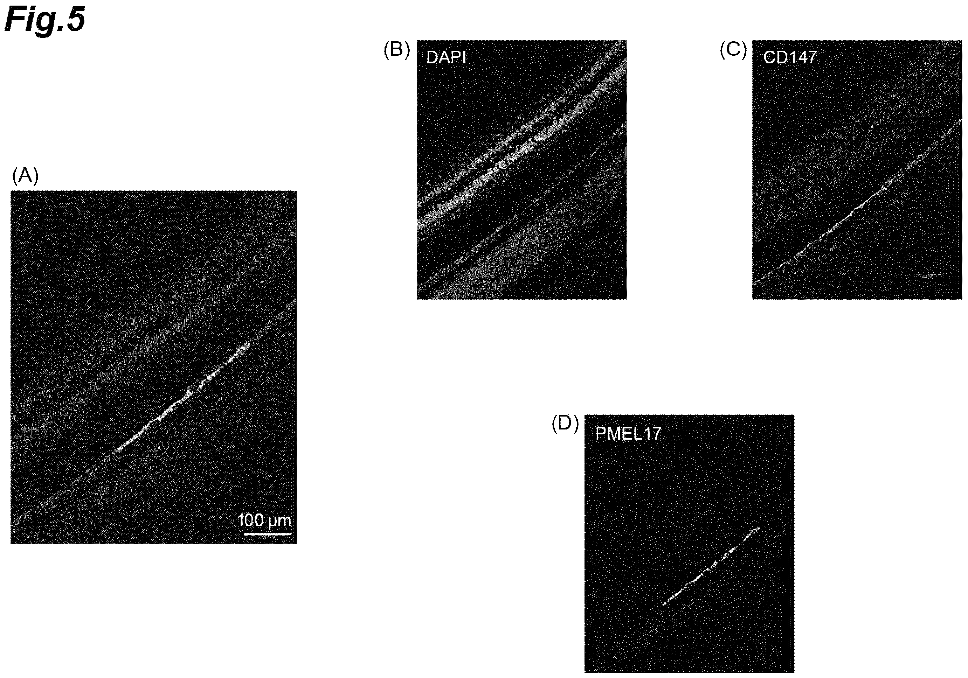

PMEL17 positive sites were observed in most of the individuals. Many of the positive sites were observed in a monolayer in the monkey retinal pigment epithelial cell layer, confirming that the injected human iPS cell-derived RPE cells were integrated into and engrafted into the monkey retinal pigment epithelial layer. Representative low-magnification fluorescence immunostaining images of the 100-fold diluted PROVISC group (Individual No. 1, 4 weeks after surgery) are shown in Figures 3 and 4. Figure 3 (A) is an image of each stain superimposed, Figure 3(B) shows the results of DAPI staining only, Figure 4(A) shows the results of CD147 staining only, and Figure 4(B) shows the results of PMEL17 staining. The sites indicated by the arrows in Figure 3(A) represent sites of consecutive RPE cell engraftment, and five sites of consecutive engraftment in this individual (Individual No. 1) were identified as Grade 3. Figure 5 shows high-magnification fluorescent immunostaining images of Individual No. 1 (4 weeks after surgery), where Figure 5(A) is an image of each stain superimposed, and Figure 5(B) to (D) are images of each single stain.

Engraftment was scored for the hyaluronic acid-supplemented medium (100-fold dilution) group, which showed good inhibition of cell leakage. In addition, the score per unit cell number was calculated because the dose volume (total number of cells injected) varied from individual to individual. The results are shown in Table 3. It was confirmed from Table 3 that the PROVISC condition resulted in wide-range engraftment of the injected cells, whereas the VISCOAT condition resulted in engraftment at only a few sites.

**[Table 3]**

| Individual No. | Transplantation medium | Suspension concentration (cell/mL) | Dose volume (µL) | Observation period (week) | Number of slices with grade ≥ 1 | Total score | Score/ 10⁵ cells |
|---|---|---|---|---|---|---|---|
| 1 | 100-fold diluted PROVISC | 5×10^6 | 60 | 4 | 8 | 20 | 6.67 |
| 2 | | 5×10^6 | 80 | 4 | 10 | 21 | 5.25 |
| 3 | 100-fold diluted VISCOAT | 5×10^6 | 50 | 4 | 2 | 3 | 1.20 |
| 4 | | 5×10^6 | 100 | 4 | 3 | 3 | 0.60 |

Based on the above, it was found that the transplantation medium containing sodium hyaluronate with an average molecular weight of 1.5 to 3.9 million at a specific concentration is the most suitable for preventing the leakage of the injected cells into the vitreous cavity and achieving wide-range engraftment.

### <Example 3: Viscosity Measurement of Transplantation Medium>

The following media were prepared as transplantation media. Based on the results of Examples 1 and 2, the media (2) to (7) were suitable transplantation media, and among them, (3) and (6), especially (6), were preferred.

(1) Hank's balanced salt solution HBSS(+)
(2) 20-Fold diluted VISCOAT (HBSS(+):VISCOAT = 20:1)
(3) 100-Fold diluted VISCOAT (HBSS(+):VISCOAT = 100:1)
(4) 200-Fold diluted VISCOAT (HBSS(+):VISCOAT = 200:1)
(5) 20-Fold diluted PROVISC (HBSS(+):PROVISC = 20:1)
(6) 100-Fold diluted PROVISC (HBSS(+):PROVISC = 100:1)
(7) 200-Fold diluted PROVISC (HBSS(+):PROVISC = 200:1)

The viscosities of the media (1) to (7) above were measured using a viscosity measuring device under the conditions of 25°C and a shear rate of 1/s = 2. The results are shown in Table 4.

**[Table 4]**

| Medium | Viscosity (mPa·s) | Concentration of sodium hyaluronate (w/v) | Concentration of chondroitin sulfate sodium (w/v) |
|---|---|---|---|
| (1)HBSS(+) | 0.829 | N.A | N.A |
| (2) HBSS(+)/VISCOAT (20:1) | 4.38 | 0.15% | 0.20% |
| (3) HBSS(+)/VISCOAT (100:1) | 2.23 | 0.03% | 0.04% |
| (4) HBSS(+)/VISCOAT (200:1) | 1.17 | 0.015% | 0.02% |
| (5) HBSS(+)/PROVISC (20:1) | 5.78 | 0.05% | N.A |
| (6) HBSS(+)/PROVISC (100:1) | 2.13 | 0.01% | N.A |
| (7) HBSS(+)/PROVISC (200:1) | 1.25 | 0.005% | N.A |

| | | | |
|---|---|---|---|
| N.A.: substantially free | | | |

The correlation between rate of shear (shear rate) and viscosity of the media (1) to (7) was measured under the condition of 25°C using a viscosity measuring device. The results are shown in Table 5. As a result, it was found that among the media (1) to (7), the amount of change in viscosity of the media (3) and (6) was larger than that of the other media.

**[Table 5]**

| Medium | Viscosity at each shear rate (1/s) (mPa·s) | | | | | Viscosity difference* (mPa·s) |
|---|---|---|---|---|---|---|
| | 1 | 2 | 10 | 100 | 1000 | |
| (1)HBSS(+) | 0.981 | 0.829 | 0.865 | 0.890 | 0.909 | 0.12 |
| (2) HBSS(+)/VISCOAT (20:1) | 4.45 | 4.38 | 4.37 | 4.36 | 3.97 | 0.08 |
| (3) HBSS(+)/VISCOAT (100:1) | 2.68 | 2.23 | 1.96 | 1.71 | 1.31 | 0.72 |
| (4) HBSS(+)/VISCOAT (200:1) | 1.35 | 1.17 | 1.09 | 1.08 | 1.07 | 0.26 |
| (5) HBSS(+)/PROVISC (20:1) | 5.87 | 5.78 | 5.63 | 4.66 | 3.07 | 0.24 |
| (6) HBSS(+)/PROVISC (100:1) | 3.13 | 2.13 | 1.89 | 1.66 | 1.25 | 1.24 |
| (7) HBSS(+)/PROVISC (200:1) | 1.30 | 1.25 | 1.16 | 1.14 | 1.07 | 0.14 |

| | | | | | | |
|---|---|---|---|---|---|---|
| *Absolute value of difference in viscosity (mPa·s) between viscosity at shear rate 1 (1/s) and viscosity at shear rate 10 (1/s) | | | | | | |

## Claims

1. A transplantation medium for subretinal transplantation of a retinal pigment epithelial cell, the transplantation medium comprising:
hyaluronic acid and a pharmaceutically acceptable aqueous liquid,
wherein a viscosity at a shear rate of 2 (1/s) at 25°C is 1.5 to 4 mPa·s.

2. The transplantation medium according to claim 1, wherein the viscosity at a shear rate of 2 (1/s) at 25°C is 2 to 3 mPa·s.

3. The transplantation medium according to claim 1 or 2, wherein the viscosity at a shear rate of 1000 (1/s) at 25°C is 3 mPa·s or less, and a difference in viscosity between the viscosity at a shear rate of 1 (1/s) and the viscosity at a shear rate of 10 (1/s) is 0.5 to 1.5 mPa·s.

4. The transplantation medium according to any one of claims 1 to 3, wherein pH is 7.0 to 7.5.

5. The transplantation medium according to any one of claims 1 to 4, wherein the pharmaceutically acceptable aqueous liquid is a balanced salt solution.

6. The transplantation medium according to any one of claims 1 to 5, which is free of an antibacterial agent and a preservative.

7. The transplantation medium according to any one of claims 1 to 6, which is free of chondroitin sulfate.

8. The transplantation medium according to any one of claims 1 to 7, wherein in the transplantation, the vitreous body is replaced with air after subretinal transplantation.

9. A transplantation composition comprising:
retinal pigment epithelial cells for transplantation; and
the transplantation medium according to any one of claims 1 to 8.

10. The transplantation composition according to claim 9, wherein the retinal pigment epithelial cells for transplantation is comprised at a concentration of 1 × 10⁶ cells/mL to 5 × 10⁷ cells/mL.

11. The transplantation composition according to claim 9 or 10, wherein a volume per transplantation is 10 µL to 100 µL.
